(19) Europäisches Patentamt
European Patent Office
Office européen des brevets

(11) Veröffentlichungsnummer : **0 021 231**
**B1**

(12) **EUROPÄISCHE PATENTSCHRIFT**

(45) Veröffentlichungstag der Patentschrift : **05.10.83**

(51) Int. Cl.³ : **C 07 H 19/06**, C 07 H 19/08// C07H15/04

(21) Anmeldenummer : **80103219.4**

(22) Anmeldetag : **10.06.80**

(54) **Verfahren zur Herstellung von 5'-Deoxy-5-fluoruridin und Zwischenprodukte in diesem Verfahren.**

(30) Priorität : **15.06.79 CH 5625/79**

(43) Veröffentlichungstag der Anmeldung : **07.01.81 Patentblatt 81/01**

(45) Bekanntmachung des Hinweises auf die Patenterteilung : **05.10.83 Patentblatt 83/40**

(84) Benannte Vertragsstaaten : **AT BE CH DE FR GB IT LI LU NL SE**

(56) Entgegenhaltungen :
**DE A 1 812 268**
**FR A 1 489 919**
**US A 4 071 680**
**ANGEWANDTE CHEMIE, International Ed. in English, Band 14, Nr. 5, Mai 1975, Verlag Chemie GmbH, Academic Press Inc. H. VORBRUGGEN et al. « New catalysts for the synthesis of nucleosides », Seiten 421-422**
**CHEMICAL ABSTRACTS, Band 77, Nr. 19. 6. November 1972, Seite 434, Zusammenfassung 126977g. Columbus, Ohio, USA HANESSIAN, S. et al. « Procedures for the direct replacement of primary hydroxyl groups in carbohydrates by halogen »**

(73) Patentinhaber : **F. HOFFMANN-LA ROCHE & CO. Aktiengesellschaft**
**CH-4002 Basel (CH)**

(72) Erfinder : **D'Sousa, Richard**
**Peter Rotstrasse 22**
**CH-4058 Basel (CH)**
Erfinder : **Kiss, Joseph, Prof. Dr.**
**Hangstrasse 9**
**CH-4144 Arlesheim (CH)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

**EP 0 021 231 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Herstellung von 5'-Deoxy-5-fluoruridin und Zwischenprodukte in diesem Verfahren

5'-Deoxy-5-fluoruridin (I) ist eine bekannte Verbindung (z. B. aus U.S.P. 4,071,680) mit sehr guter cytostatischer Aktivität, die in einer mehrstufigen Synthese aus 5-Fluoruridin erhältlich ist, wobei die Eliminierung der 5'-Hydroxygruppe über die entsprechende 5'-Jodverbindung erfolgt.

Die vorliegende Erfindung betrifft ein neues Verfahren zur Herstellung von 5'-Deoxy-5-fluoruridin in sehr guter Ausbeute und einfacher Weise über 5-Deoxy-1,2,3-tri-O-acyl-D-ribofuranosid (III) und 1-(5-Deoxy-2,3-di-O-acyl-β-D-ribofuranosyl)-5-fluoruracil (II). Die Verbindung der Formel III ist leicht aus D-Ribose bzw. aus dem bekannten und aus D-Ribose leicht zugänglichen Methyl-2,3-O-isopropyliden-D-ribofuranosid (VII) erhältlich.

Das erfindungsgemässe Verfahren ist dadurch gekennzeichnet, dass man

a) Methyl-2,3-O-isopropyliden-D-ribofuranosid bei Raumtemperatur in Methylenchlorid und in Gegenwart von Triphenylphosphin mit Brom behandelt,

b) das in dem erhaltenen Methyl-(5-deoxy-5-brom-2,3-O-isopropyliden)-D-ribofuranosid enthaltene Bromatom reduktiv eliminiert,

c) das erhaltene Methyl-(5-deoxy-2,3-O-isopropyliden)-D-ribofuranosid entweder zu 5-Deoxy-D-ribose hydrolysiert oder einer selektiven Abspaltung der Isopropylidengruppe unterwirft,

d) das Produkt von Stufe c) acyliert,

e) das Produkt von Stufe d), ein 5-Deoxy-1,2,3-tri-O-acyl-D-ribofuranosid oder ein 5-Deoxy-2,3-di-O-acyl-1-O-methyl-D-ribofuranosid, mit 2,4-Bis-(trimethylsilyl)-5-fluoruracil umsetzt und das β-Anomere, 1-(5-Deoxy-2,3-di-O-acyl-β-D-ribofuranosyl)-5-fluoruracil aus dem Reaktionsgemisch abtrennt, und

f) die Acylgruppen des erhaltenen 1-(5-Deoxy-2,3-di-O-acyl-β-D-ribofuranosyl)-5-fluoruracils abspaltet.

Als Acylgruppen kommen die üblichen in der Zuckerchemie als Schutzgruppen bekannten Reste aliphatischer und aromatischer Säuren in Frage, wobei die Benzoyl- und Acetylgruppe bevorzugt sind. Die Spaltung (Stufe f) verläuft in an sich bekannter Weise, praktisch quantitativ, und kann durch saure oder alkalische Hydrolyse erfolgen, beispielsweise in wässrigem oder alkoholischem Medium mittels einer Mineralsäure oder einer starken organischen Säure. Vorzugsweise wird die Hydrolyse in alkoholischem, wasserfreiem Medium, z. B. in Methanol, durch Behandlung mit einem Alkalimetallalkoholat, z. B. Natriummethylat, durchgeführt (Methode von Zemplén). Damit die Spaltung quantitativ verläuft, muss das sich bildende Methylacetat kontinuierlich oder durch wiederholte Vakuumdestillation entfernt werden. Die Abspaltung der Acylreste kann aber auch durch Behandlung mit einer starken organischen Base, z. B. einem Trialkylamin, wie Triethylamin, in einem organischen Lösungsmittel, vorzugsweise Methanol, oder mittels eines stark basischen Ionenaustauschers ($OH^-$-Form) erfolgen.

Die Aufarbeitung des Reaktionsgemisches kann in der üblichen Weise geschehen. Vorteilhafterweise wird die Neutralisation des Gemisches durch Behandlung mit einem Kationenaustauscher ($H^+$-Form) erreicht; aus dieser Lösung kann nach Entfernung des Austauscherharzes das 5'-Deoxy-5-fluoruridin durch Einengen in hoher Ausbeute und Reinheit erhalten werden.

Die Verbindungen der Formel II, sind neu und ebenfalls Gegenstand der vorliegenden Erfindung. Sie können dadurch hergestellt werden (Stufe e) dass man ein 5-Deoxy-1,2,3-tri-O-acyl-D-ribofuranosid (IIIa) oder ein 5-Deoxy-2,3-di-O-acyl-1-O-methyl-D-ribofuranosid (IIIb) in Form des Anomerengemisches mit 2,4-Bis-(trimethylsilyl)-5-fluoruracil (IV) in Gegenwart eines geeigneten Katalysators, vorzugsweise Trimethylsilyl-trifluormethansulfonat oder Zinntetrachlorid, bei Raumtemperatur oder darunter, vorzugsweise unter Eiskühlung, in einem inerten organischen Lösungsmittel, umsetzt und aus dem Reaktionsgemisch das gewünschte β-Anomere isoliert. Im Fall der 2,3-Di-O-acetylverbindung (II) verläuft diese Auftrennung besonders einfach und praktisch quantitativ, da das β-Anomere selbst in heissem Ethylacetat sehr schwer löslich ist.

Die Verbindung IIIa kann aus Methyl-(5-deoxy-2,3-O-isopropyliden)-D-ribofuranosid (V) dadurch erhalten werden (Stufe c), dass man letzteres durch Hydrolyse mit einer wässrigen Mineralsäure in bekannter Weise in 5-Deoxy-D-ribose überführt und diese in ebenfalls bekannter Weise acyliert, vorzugsweise acetyliert, was z. B. mit Acetanhydrid in Pyridin geschehen kann. Die Reinigung kann entweder durch Destillation oder, vorzugsweise, an Aluminiumoxid oder Kieselgel erfolgen. Die Verbindung IIIb kann ebenfalls aus Verbindung V erhalten werden und zwar durch selektive Abspaltung der Isopropylidengruppe, z. B. durch 10 minütige Behandlung mit einer verdünnten Säure (0,01 N HCl) und anschliessende Acylierung (z. B. Acetanhydrid/Pyridin).

Andererseits kann aber eine Verbindung II auch aus der Verbindung V dadurch erhalten werden, dass man letztere in an sich bekannter Weise durch selektive Abspaltung der Isopropylidengruppe und anschliessende Acylierung in ein Methyl-(5-deoxy-2,3-di-O-acyl)-D-ribofuranosid überführt und dieses dann in der oben beschriebenen Weise mit 2,4-Bis-(trimethylsilyl)-5-fluoruracil umsetzt. Diese Verfahrensroute erbringt jedoch eine vergleichsweise geringere Ausbeute an Verbindung II.

Die Herstellung der Verbindung V kann in an sich bekannter Weise aus dem bekannten Methyl-2,3-O-isopropyliden-D-ribofuranosid (VII) erfolgen und zwar durch Ersatz der 5-Hydroxygruppe gegen Brom (Verbindung VI) und anschliessende reduktive Eliminierung des Bromatoms.

Für die erste der beiden Reaktionen wird jedoch erfindungsgemäß eine neue Methode benutzt (Stufe

a), die darin besteht, dass das Methyl-2,3-O-isopropyliden-D-ribofuranosid bei Raumtemperatur in Methylenchlorid und in Gegenwart von Triphenylphosphin mit Brom behandelt wird. Bei einfacher Reaktionsführung wird auf diese Weise ein sehr reines Produkt in hoher Ausbeute erhalten, sofern unter striktem Wasserausschluss gearbeitet wird.

Das erhaltene Methyl-(5-deoxy-5-brom-2,3-O-isopropyliden)-D-ribofuranosid (VI) kann leicht (Stufe b) in die entsprechende 5-Deoxyverbindung (V) übergeführt werden durch katalytische Hydrierung in einem protischen Lösungsmittel, beispielsweise einem Alkohol, vorzugsweise Methanol, unter Verwendung eines Edelmetallkatalysators, z. B. Palladium, ggf. auf einem inerten Trägermaterial, wie Kohle oder Bariumsulfat, oder auch in Gegenwart von Raney-Nickel. Die Hydrierung kann bei einer Temperatur zwischen 0 und 60 °C, vorzugsweise bei Zimmertemperatur, unter einem Druck von 1 bis 5 Atmosphären, vorzugsweise unter Normaldruck, in Gegenwart einer anorganischen oder organischen Base, wie Kaliumhydroxid oder Triäthylamin, erfolgen.

Das folgende Reaktionsschema gibt eine Uebersicht über die vorstehend beschriebene Erfindung, die durch die ebenfalls folgenden Beispiele illustriert wird.

Reaktionsschema :

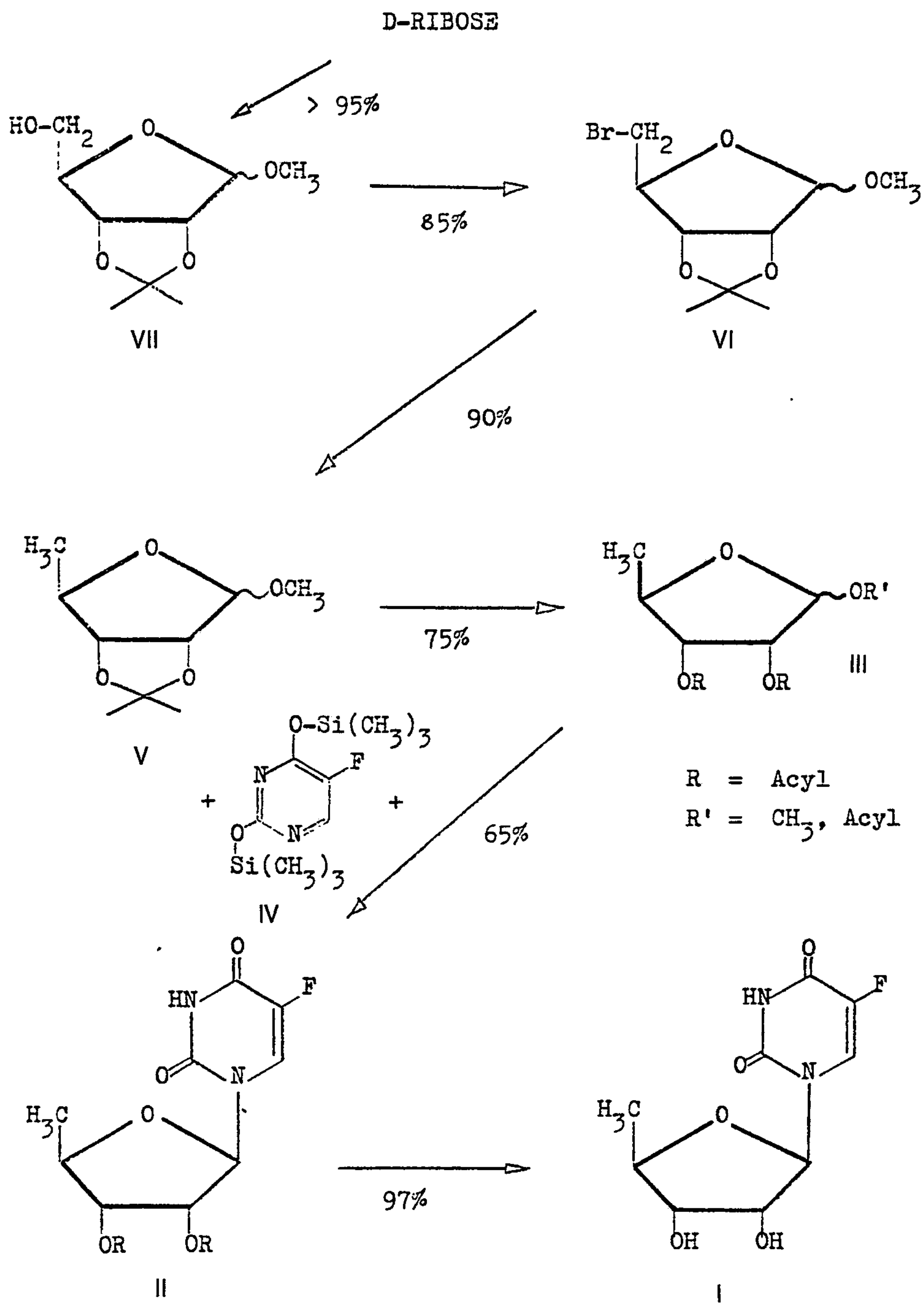

VII VI V IV III II I

Beispiel 1

a) Eine Lösung von 204,22 g Methyl-2,3-O-isopropyliden-D-ribofuranosid und 524,5 g Triphenylphosphin in 7 000 ml Methylenchlorid wurde auf 5-8 °C gekühlt und innerhalb von 60 Minuten unter Rühren mit 102 ml Brom versetzt. Das Reaktionsgemisch wurde 4 Stunden unter Rückfluss erhitzt und anschliessend 14 Stunden bei Zimmertemperatur gerührt. Nach Zusatz von 50 ml Methanol wurde viermal mit je 2 000 ml Wasser, mit 2 000 ml 3 %iger wässriger $NaHCO_3$-Lösung und nochmals mit 2 000 ml Wasser gewaschen. Die organische Phase wurde über wasserfreiem Natriumsulfat getrocknet und unter vermindertem Druck und leichtem Erwärmen auf etwa 1 000 ml eingeengt. Die konzentrierte Lösung wurde mit 1 000 ml Hexan versetzt, die gebildeten Kristalle wurden abgesaugt und zweimal mit je 300 ml Hexan gewaschen. Der aus den vereinigten Lösungen durch Eindampfen erhaltene Rückstand wurde zweimal mit je 1 000 ml Hexan extrahiert und schliesslich an einer Kieselgelsäule (6 × 80 cm) mittels 1 500 ml Hexan eluiert. Die vereinigten Hexan-Lösungen lieferten nach Einengen im Vakuum und anschliessender Vakuumdestillation 226 g (85 %) Methyl-(5-deoxy-5-brom-2,3-O-isopropyliden)-D-ribofuranosid, $Kp._{0,6}$ = 72-75 °C, $n_D^{24}$ = 1,471 2, $[\alpha]_D^{364} = -237,6°$ (1 % in $CHCl_3$).

b) 802 g Methyl-(5-deoxy-5-brom-2,3-O-isopropyliden)-D-ribofuranosid in 7 500 ml Methanol wurden bei Raumtemperatur in Gegenwart von methanolischem Kaliumhydroxid (185 g in 3 000 ml Methanol) und 50 g 5 %igem Pd/C 15 Stunden hydriert. Das Reaktionsgemisch wurde nach Filtration unter vermindertem Druck bei 35° eingeengt, der Rückstand mit 2 000 ml Hexan verdünnt und auf eine Kieselgelsäule (5 × 80 cm) gegeben, die mit 4 000 ml Hexan eluiert wurde. Nach Eidampfen des Eluates und Vakuumdestillation wurden 510 g (90 %) Methyl-(5-deoxy-2,3-O-isopropyliden)-D-ribofuranosid, $Kp._{10-12}$ 72-75 °C, $n_D^{20}$ = 1,434 0, $[\alpha]_D^{25} = -96,6°$ (c = 0,34 in $CHCl_3$), erhalten.

c) Ein Gemisch aus 282 g Methyl-(5-deoxy-2,3-O-isopropyliden)-D-ribofuranosid, 162 ml 1 N Salzsäure und 3 860 ml Wasser wurde 2 Stunden unter Rühren auf 110 °C erwärmt. Durch Eingiessen in Eiswasser wurde die Lösung auf 16-20 °C abgekühlt und durch Zusatz von 1 000 ml des Anionenaustauschers Amberlite® IR-45 ($OH^-$-Form) neutralisiert. Nach 30 minütigem Rühren lag der pH-Wert des Gemisches bei 6. Der Ionenaustauscher wurde abfiltriert und mit 2 000 ml Wasser gewaschen ; die wässrigen Lösungen wurden unter vermindertem Druck bei 40-45 °C eingeengt und der ölige Rückstand in Gegenwart von 3 000 ml Pyridin eingedampft. Die erhaltene rohe 5-Deoxy-D-ribose (202 g) wurde in 3 000 ml Pyridin gelöst und durch Behandlung mit 720 g Acetanhydrid acetyliert. Das Reaktionsgemisch wurde 20 Stunden bei Raumtemperatur stehengelassen, unter vermindertem Druck auf ein Volumen von 400-500 ml eingeengt und unter Rühren in 2 000 ml einer gesättigten wässrigen $NaHCO_3$-Lösung eingegossen. Die wässrige Lösung wurde zweimal mit je 1 000 ml Methylenchlorid extrahiert, der Extrakt wurde mit Wasser gewaschen (2 × 1 000 ml), über wasserfreiem Natriumsulfat getrocknet und unter vermindertem Druck eingeengt. Der sirupöse Rückstand wurde zweimal nach Zusatz von jeweils 2 000 ml Toluol eingedampft, in 1 000 ml Ether aufgenommen und 15 Minuten mit 10 g Aktivkohle gerührt. Nach Filtration und Waschen des Rückstandes mit 500 ml Ether wurde die etherische Phase eigedampft und durch Vakuumdestillation gereinigt ($Kp._{0,1-0,3}$ = 96-102 °C). Es wurden 210 g (75 %) 5-Deoxy-1,2,3-tri-O-acetyl-D-ribofuranosid als Anomerengemisch erhalten. Das daraus isolierte kristalline β-Anomere hat einen Schmelzpunkt von 66-67 °C.

d) Eine Lösung von 305 g frisch destilliertem 5-Deoxy-1,2,3-tri-O-acetyl-D-ribofuranosid (Anomerengemisch) in 3 000 ml Methylenchlorid wurde mit aus 165 g 5-Fluoruracil und 620 ml Hexamethyldisilazan frisch hergestelltem 2,4-Bis-(trimethylsilyl)-5-fluoruracil in 1 000 ml Methylenchlorid versetzt. Die Umsetzung fand unter Eiskühlung und kräftigem Rühren in Gegenwart von 230 ml Trimethylsilyltrifluormethansulfonat in 250 ml Methylenchlorid statt. Das Gemisch wurde 20 Stunden bei Raumtemperatur stehengelassen, in 3 000 ml gesättigte wässrige $NaHCO_3$-Lösung eingegossen, 30 Minuten gerührt und dreimal mit je 800 ml Methylenchlorid extrahiert. Die Extrakte wurden dreimal mit je 300 ml Wasser gewaschen, über wasserfreiem Natriumsulfat getrocknet und unter vermindertem Druck auf ein Volumen von 500-600 ml eingeengt. Das Konzentrat wurde mit 500 ml Hexan versetzt und die Lösung bei Raumtemperatur innerhalb von etwa 2 Stunden eingedampft. Das erhaltene kristalline Produkt wurde mit einer auf 0 °C gekühlten Mischung aus 900 ml Ethylacetat und 900 ml Hexan gewaschen und 10 Minuten mit 500 ml Ethylacetat unter Rückfluss erhitzt. Die Lösung wurde abgekühlt und 12 Stunden im Kühlschrank stehengelassen. Es wurden 250 g reines 1-(5-Deoxy-2,3-di-O-acetyl-β-D-ribofuranosyl)-5-fluoruracil, F. 176-177 °C, erhalten.

e) Einer Lösung von 860 g 1-(5-Deoxy-2,3-di-O-acetyl-β-D-ribofuranosyl)-5-fluoruracil in 16 l Methanol wurde aus 42 g Natrium und 2 000 ml Methanol frisch hergestelltes Nariummethoxid zugesetzt. Das während der Reaktion entstehende Methylacetat wurde durch mehrmaliges Einengen des Reaktionsgemisches entfernt. Das Gemisch wurde durch Zusatz von 1 000 ml des Kationenaustauscherharzes Amberlite® IRC-120 ($H^+$-Form), frisch gewaschen mit Methanol, neutralisiert. Nach Entfernung des Austauscherharzes wurde die methanolische Lösung unter vermindertem Druck eingeengt und das kristallin anfallende Endprodukt mit einem auf 0 °C gekühlten Gemisch aus 1 000 ml Methanol und 1 000 ml Ethylacetat gewaschen. Durch Aufarbeitung der Mutterlaugen wurden insgesamt 680 g (97 %) reines 5'-Deoxy-5-fluoruridin, F. 192-193 °C, $[\alpha]_D^{25} = +139°$ (c = 0,9 in Methanol), erhalten.

Beispiel 2

Eine Lösung von 100 g Methyl-(5-deoxy-2,3-O-isopropyliden)-D-ribofuranosid in 500 ml Methanol wurde mit 500 ml 0,1N Schwefelsäure versetzt und solange auf dem Wasserbad unter Rückfluss erhitzt, bis dünnschichtchromatographisch kein Ausgangsmaterial mehr nachgewiesen werden konnte. Das Reaktionsgemisch wurde dann unter Rühren durch Zusatz von Bariumcarbonat neutralisiert (pH 6-7), über eine Glasfritte abgesaugt und das Filtrat unter vermindertem Druck zur Trockene eingeengt. Der Rückstand wurde zweimal mit einem Gemisch aus 150 ml Methanol und 100 ml Benzol aufgenommen und wieder eingeengt und schliesslich in 500 ml trockenem Pyridin und 400 ml Acetanhydrid aufgenommen. Nach 24 stündigem Stehen bei 20-22 °C wurde unter vermindertem Druck eingeengt, zweimal jeweils mit 200 ml Toluol aufgenommen und eingeengt und der Rückstand in 300 ml Methylenchlorid gelöst. Die Lösung wurde zweimal mit je 150 ml 1N Schwefelsäure und mit 100 ml 2 %iger Natriumbicarbonatlösung gewaschen, über Natriumsulfat getrocknet und unter vermindertem Druck eingeengt. Es wurden 130 g rohes Methyl-(5-deoxy-2,3-di-O-acetyl)-D-ribofuranosid in Form eines gelblichen, öligen Materials erhalten, das unter vermindertem Druck destilliert wurde : $Kp._{0.3}$ 78-79 °C, $n_D^{20}$ = 1.437 1-1.438 0.

Zu einer Lösung von 8,1 g frisch destilliertem Methyl-(5-deoxy-2,3-di-O-acetyl)-D-ribofuranosid in 81 ml Methylenchlorid wurden 30 ml einer Lösung von Bis-trimethylsilyl-5-fluoruracil (hergestellt aus 5,2 g Fluoruracil und 20 ml Hexamethyldisilazan) in Methylenchlorid gegeben. Das Reaktionsgemisch wurde eisgekühlt und unter Rühren mit einer Lösung von 7 ml Trimethylsilyl-trifluormethansulfonat in 8 ml Methylenchlorid versetzt. Die Mischung wurde 20 Stunden bei Raumtemperatur stehengelassen, in 200 ml gesättigte wässrige Natriumbicarbonatlösung gegosen, 30 Minuten gerührt und das Gemisch dreimal mit je 100 ml Methylenchlorid extrahiert. Die vereinigten Extrakte wurden zweimal mit je 50 ml Wasser gewaschen, über wasserfreiem Natriumsulfat getrocknet, ohne Erhitzen unter vermindertem Druck auf 60 ml eingeengt und nach Zusatz von 60 ml Hexan unter den gleichen Bedingungen eingeengt. Der Rückstand wurde 4 Stunden im Kühlschrank stehengelassen, die Kristalle wurden abfiltriert, mit einem kalten 1 : 1-Gemisch von Ethylacetat und Hexan und schliesslich mit Ethylacetat gewaschen. Es wurden 7 g reines 1-(5-Deoxy-2,3-di-O-actyl-β-D-ribofuranosyl)-5-fluoruracil, F. 179-181 °C, $[\alpha]_D^{20}$ = + 34,6° (c = 1.0 in Methanol), erhalten.

2,5 g 1-(5-Deoxy-2,3-di-O-acetyl-β-D-ribofuranosyl)-5-fluoruracil wurden in 60 ml Methanol in Gegenwart von 0,3 ml Triethylamin so lange unter Rückfluss erhitzt, bis dünnschichtchromatographisch kein Ausgangsprodukt mehr nachgewiesen werden konnte. Die farblose, klare Lösung wurde unter vermindertem Druck eingeengt und das erhaltene weisse, kristalline Material wurde aus Wasser umkristallisiert. Es wurde reines 5'-Deoxy-5-fluoruridin, F. 192-193 °C, erhalten (Ausbeute 98 %).

**Ansprüche** (für die Vertragsstaaten : BE, CH, DE, FR, GB, IT, LI, LU, NL, SE)

1. Verfahren zur Herstellung von 5'-Deoxy-5-fluoruridin, dadurch gekennzeichnet, dass man

a) Methyl-2,3-O-isopropyliden-D-ribofuranosid bei Raumtemperatur in Methylenchlorid und in Gegenwart von Triphenylphosphin mit Brom behandelt,

b) das in dem erhaltenen Methyl-(5-deoxy-5-brom-2,3-O-isopropyliden)-D-ribofuranosid enthaltene Bromatom reduktiv eliminiert,

c) das erhaltene Methyl-(5-deoxy-2,3-O-isopropyliden)-D-ribofuranosid entweder zu 5-Deoxy-D-ribose hydrolysiert oder einer selektiven Abspaltung der Isopropylidengruppe unterwirft,

d) das Produkt von Stufe c) acyliert,

e) das Produkt von Stufe d), ein 5-Deoxy-1,2,3-tri-O-acyl-D-ribofuranosid oder ein 5-Deoxy-2,3-di-O-acyl-1-O-methyl-D-ribofuranosid, mit 2,4-Bis-(trimethylsilyl)-5-fluoruracil umsetzt und das β-Anomere, 1-(5-Deoxy-2,3-di-O-acyl-β-D-ribofuranosyl)-5-fluoruracil aus dem Reaktionsgemisch abtrennt, und

f) die Acylgruppen des erhaltenen 1-(5-Deoxy-2,3-di-O-acyl-β-D-ribofuranosyl)-5-fluoruracils abspaltet.

2. Verfahren gemäss Anspruch 1, dadurch gekennzeichnet, dass man in Stufe d) 5-Deoxy-D-ribose zu 5-Deoxy-1,2,3-tri-O-acetyl-D-ribofuranosid acetyliert.

3. Ein 1-(5-Deoxy-2,3-di-O-acyl-β-ribofuranosyl)-5-fluoruracil, in dem die Acylgruppe ein in der Zuckerchemie als Schutzgruppe üblicher Rest einer aliphatischen oder aromatischen Säure ist.

4. 1-(5-Deoxy-2,3-di-O-acetyl-β-D-ribofuranosyl)-5-fluoruracil.

**Ansprüche** (für der Vertragsstaat AT)

1. Verfahren zur Herstellung von 5'-Deoxy-5-fluoruridin, dadurch gekennzeichnet, dass man

a) Methyl-2,3-O-isopropyliden-D-ribofuranosid bei Raumtemperatur in Methylenchlorid und in Gegenwart von Triphenylphosphin mit Brom behandelt,

b) das in dem erhaltenen Methyl-(5-deoxy-5-brom-2,3-O-isopropyliden)-D-ribofuranosid enthaltene Bromatom reduktiv eliminiert,

c) das erhaltene Methyl-(5-deoxy-2,3-O-isopropyliden)-D-ribofuranosid entweder zu 5-Deoxy-D-ribose hydrolysiert oder einer selektiven Abspaltung der Isopropylidengruppe unterwirft,

d) das Produkt von Stufe c) acyliert,

e) das Produkt von Stufe d), ein 5-Deoxy-1,2,3-tri-O-acyl-D-ribofuranosid oder ein 5-Deoxy-2,3-di-O-acyl-1-O-methyl-D-ribofuranosid, mit 2,4-Bis-(trimethylsilyl)-5-fluoruracil umsetzt und das β-Anomere, 1-(5-Deoxy-2,3-di-O-acyl-β-D-ribofuranosyl)-5-fluoruracil aus dem Reaktionsgemisch abtrennt, und

f) die Acylgruppen des erhaltenen 1-(5-Deoxy-2,3-di-O-acyl-β-D-ribofuranosyl)-5-fluoruracils abspaltet.

2. Verfahren gemäss Anspruch 1, dadurch gekennzeichnet, dass man in Stufe d) 5-Deoxy-D-ribose zu 5-Deoxy-1,2,3-tri-O-acetyl-D-ribofuranosid acetyliert.

**Claims** (for the Contracting States : BE, CH, DE, FR, GB, IT, LI, LU, NL, SE)

1. Process for the manufacture of 5'-deoxy-5-fluorouridine, characterized in that

a) methyl 2,3-O-isopropylidene-D-ribofuranoside is treated with bromine at room temperature in methylene chloride and in the presence of triphenylphosphine,

b) the bromine atom present in the resulting methyl (5-deoxy-5-bromo-2,3-O-isopropylidene)-D-ribofuranoside is reductively eliminated,

c) the resulting methyl (5-deoxy-2,3-O-isopropylidene)-D-ribofuranoside is either hydrolyzed to 5-deoxy-D-ribose or is subjected to a selective cleavage of the isopropylidene group,

d) the product of step c) is acylated,

e) the product of step d), a 5-deoxy-1,2,3-tri-O-acyl-D-ribofuranoside or a 5-deoxy-2,3-di-O-acyl-1-O-methyl-D-ribofuranoside, is reacted with 2,4-bis-(trimethylsilyl)-5-fluorouracil and the β-anomer, 1-(5-deoxy-2,3-di-O-acyl-β-D-ribofuranosyl)-5-fluorouracil, is separated from the reaction mixture, and

f) the acyl groups of the resulting 1-(5-deoxy-2,3-di-O-acyl-β-D-ribofuranosyl)-5-fluorouracil are cleaved off.

2. Process in accordance with claim 1, characterized in that in step d) 5-deoxy-D-ribose is acetylated to 5-deoxy-1,2,3-tri-O-acetyl-D-ribofuranoside.

3. A 1-(5-deoxy-2,3-di-O-acyl-β-ribofuranosyl)-5-fluorouracil in which the acyl group is the residue of an aliphatic or aromatic acid which is customary as a protecting group in sugar chemistry.

4. 1-(5-Deoxy-2,3-di-O-acetyl-β-D-ribofuranosyl)-5-fluorouracil.

**Claims** (for the Contracting State AT)

1. Process for the manufacture of 5'-deoxy-5-fluorouridine, characterized in that

a) methyl 2,3-O-isopropylidene-D-ribofuranoside is treated with bromine at room temperature in methylene chloride and in the presence of triphenylphosphine,

b) the bromine atom present in the resulting methyl (5-deoxy-5-bromo-2,3-O-isopropylidene)-D-ribofuranoside is reductively eliminated,

c) the resulting methyl (5-deoxy-2,3-O-isopropylidene)-D-ribofuranoside is either hydrolyzed to 5-deoxy-D-ribose or is subjected to a selective cleavage of the isopropylidene group,

d) the product of step c) is acylated,

e) the product of step d), a 5-deoxy-1,2,3-tri-O-acyl-D-ribofuranoside or a 5-deoxy-2,3-di-O-acyl-1-O-methyl-D-ribofuranoside, is reacted with 2,4-bis-(trimethylsilyl)-5-fluorouracil and the β-anomer, 1-(5-deoxy-2,3-di-O-acyl-β-D-ribofuranosyl)-5-fluorouracil, is separated from the reaction mixture, and

f) the acyl groups of the resulting 1-(5-deoxy-2,3-di-O-acyl-β-D-ribofuranosyl)-5-fluorouracil are cleaved off.

2. Process in accordance with claim 1, characterized in that in step d) 5-deoxy-D-ribose is acetylated to 5-deoxy-1,2,3-tri-O-acetyl-D-ribofuranoside.

**Revendications** (pour les Etats contractants : BE, CH, LI, DE, FR, GB, IT, LU, NL, SE)

1. Procédé de préparation de la 5'-désoxy-5-fluoruridine, caractérisé en ce que :

a) on traite le méthyl-2,3-O-isopropyl-idène-D-ribofurannoside par le brome à température ambiante dans le chlorure de méthylène et en présence de triphénylphosphine,

b) on élimine par réduction l'atome de brome contenu dans le méthyl-(5-désoxy-5-bromo-2,3-O-isopropylidène)-D-ribofurannoside obtenu,

c) on soumet le méthyl-(5-désoxy-2,3-O-isopropylidène)-D-ribofurannoside obtenu soit à hydrolyse en le 5-désoxy-D-ribose, soit à une scission sélective du groupe isopropylidène,

d) on acyle le produit du stade c),

e) on fait réagir le produit du stade d), un 5-désoxy-1,2,3-tri-O-acyl-D-ribofurannoside ou un 5-désoxy-2,3-di-O-acyl-1-O-méthyl-D-ribofurannoside, avec le 2,4-bis-(triméthylsilyl)-5-fluoruracile et on

sépare l'anomère bêta, le 1-(5-désoxy-2,3-di-O-acyl-bêta-D-ribofurannoside)-5-fluoruracile du mélange de réaction, et

f) on élimine les groupes acyles du 1-(5-désoxy-2,3-di-O-acyl-bêta-D-ribofurannosyl)-5-fluoruracile obtenu.

2. Procédé selon la revendication 1, caractérisé en ce que, au stade d), on acétyle le 5-désoxy-D-ribose en 5-désoxy-1,2,3-tri-O-acétyl-D-ribofurannoside.

3. Un 1-(5-désoxy-2,3-di-O-acyl-bêta-ribofurannosyl)-5-fluoruracile dans lequel le groupe acyle est un reste d'un acide aliphatique, ou aromatique usuel en tant que groupe protecteur dans la chimie des sucres.

4. Le 1-(5-désoxy-2,3-di-O-acétyl-bêta-D-ribofurannosyl)-5-fluoruracile.

**Revendications** (pour l'Etat Contractant AT)

1. Procédé de préparation de la 5'-désoxy-5-fluoruridine, caractérisé en ce que :

a) on traite le méthyl-2,3-O-isopropylidène-D-ribofurannoside par le brome à température ambiante dans le chlorure de méthylène et en présence de triphénylphosphine,

b) on élimine par réduction l'atome de brome contenu dans le méthyl-(5-désoxy-5-bromo-2,3-O-isopropylidène)-D-ribofurannoside obtenu,

c) on soumet le méthyl-(5-désoxy-2,3-O-isopropylidène)-D-ribofurannoside obtenu soit à hydrolyse en le 5-désoxy-D-ribose, soit à une scission sélective du groupe isopropylidène,

d) on acyle le produit du stade c),

e) on fait réagir le produit du stade d), un 5-désoxy-1,2,3-tri-o-acyl-D-ribofurannoside ou un 5-désoxy-2,3-di-o-acyl-1-O-méthyl-D-ribofurannoside, avec le 2,4-bis-(triméthylsilyl)-5-fluoruracile et on sépare l'anomère bêta, le 1-(5-désoxy-2,3-di-O-acyl-bêta-D-ribofurannoside)-5-fluoruracile du mélange de réaction, et

f) on élimine les groupes acyles du 1-(5-désoxy-2,3-di-O-acyl-bêta-D-ribofurannosyl)-5-fluoruracile obtenu.

2. Procédé selon la revendication 1, caractérisé en ce que, au stade d), on acétyle le 5-désoxy-D-ribose en 5-désoxy-1,2,3-tri-O-acétyl-D-ribofurannoside.